# EUROPÄISCHE PATENTSCHRIFT

(11) **EP 2 983 587 B1**
(45) Veröffentlichungstag und Bekanntmachung des Hinweises auf die Patenterteilung: **04.10.2017**
(21) Anmeldenummer: 14710275.0
(22) Anmeldetag: 14.03.2014
(51) Int. Cl.: A61B 5/08, A61B 5/09, G01N 33/497

(54) **VERFAHREN ZUR KOMBINIERTEN ATEMGASANALYSE UND LUNGENFUNKTIONSPRÜFUNG**
METHOD FOR COMBINED RESPIRATORY GAS ANALYSIS AND LUNG FUNCTION TEST
PROCÉDÉ SERVANT À COMBINER UNE ANALYSE DES GAZ RESPIRATOIRES ET À VÉRIFIER LES FONCTIONS PULMONAIRES

(30) Priorität: 08.04.2013 DE 102013206111
(43) Veröffentlichungstag der Anmeldung: 17.02.2016
(73) Patentinhaber: Robert Bosch GmbH, 70442 Stuttgart (DE)
(72) Erfinder: LEONHARDT, Ronny, 70839 Gerlingen (DE); GIEZENDANNER-THOBEN, Robert, 70839 Gerlingen (DE); COCLICI, Cristian-Aurelian, 70195 Stuttgart (DE)
(86) Internationale Anmeldenummer: PCT/EP2014/055126
(87) Internationale Veröffentlichungsnummer: WO 2014/166697

(56) Entgegenhaltungen:
- WO-A1-2013/003429
- DE-A1-102009 038 237
- DE-A1-102009 043 236

## Beschreibung

Die vorliegende Erfindung betrifft ein Verfahren zur kombinierten Atemgasanalyse und Lungenfunktionsprüfung nach dem Oberbegriff des Anspruchs 1.

### Stand der Technik

Aus dem Stand der Technik sind Messvorrichtungen zur Unterstützung der Diagnose und Therapie von Lungenerkrankungen bekannt. In bekannter Weise werden Spirometer zur Bewertung der Lungenfunktion eingesetzt. Des Weiteren sind beispielsweise aus der WO 2004/023997 Atemgasanalyse-Geräte zur Bestimmung der NO-Konzentration in der Atemluft bekannt. Aufgrund von aus den angewandten medizinischen Analyseverfahren resultierenden Verfahrensanforderungen werden üblicherweise nur monofunktionale Spezialgeräte verwendet.
Die DE 10 2009 038 237 offenbart eine Vorrichtung zur Kombination von Atemgasanalyse und Lungenfunktionsprüfung und stellt den nächsten Stand der Technik dar. Das darin dargestellte Verfahren offenbart jedoch kein Einatmen durch eine kombinierte Einlass-/Auslassöffnung. Die DE 10 2009 043 236 beschreibt ein Multifunktionssteuerventil für Gasmessgeräte.

### Offenbarung der Erfindung

Gegenstand der vorliegenden Erfindung ist ein Verfahren zur kombinierten Atemgasanalyse und Lungenfunktionsprüfung wie in Anspruch 1 definiert. Die kombinierte Atemgasanalyse und Lungenfunktionsprüfung wird erfindungsgemäß mittels einer Vorrichtung durchgeführt, wobei die Vorrichtung folgende Merkmale umfasst:
- eine erste kombinierte Einlass-/Auslassöffnung,
- eine zweite kombinierte Einlass-/Auslassöffnung,
- eine Gasmengen-Messvorrichtung zur Bestimmung einer durchströmenden Menge des Atemgases,
- eine Gasanalysevorrichtung zur Analyse des Atemgases, und
- ein Mehrwegeventil, das zumindest zwischen einer ersten Ventilpfadkombination und einer zweiten Ventilpfadkombination verbringbar ist.
Unter einer "kombinierten Einlass-/Auslassöffnung" soll in diesem Zusammenhang insbesondere eine Öffnung der Vorrichtung verstanden werden, die in zumindest einem ersten Betriebszustand als Einlassöffnung für ein Gas und in zumindest einem zweiten Betriebszustand als Auslassöffnung für das Gas dient. Weiter definiert Anspruch 1 die Vorrichtung dadurch, dass
- mit der ersten Ventilpfadkombination des Mehrwegeventils unter Umgehung der Gasanalysevorrichtung ein erster Gaspfad zwischen der ersten kombinierten Einlass-/Auslassöffnung, der Gasmengen-Messvorrichtung und der zweiten kombinierten Einlass-/Auslassöffnung herstellbar ist, und
- mit der zweiten Ventilpfadkombination des Mehrwegeventils ein zweiter Gaspfad zwischen der ersten kombinierten Einlass-/Auslassöffnung, der Gasanalysevorrichtung, der Gasmengen-Messvorrichtung und der zweiten kombinierten Einlass-/Auslassöffnung herstellbar ist.

Mit einer solchen Ausführungsform der Vorrichtung können sowohl eine Lungenfunktionsprüfung durchgeführt als auch einzelne Atemgaskonzentrationen ermittelt werden. Mit einem Mehrwegeventil mit einer geeigneten Ausgestaltung kann zwischen zwei Gaspfaden geschaltet werden, wodurch sich Spirometrie und Gasanalysemessungen vereinen lassen. Dadurch kann eine Ermittlung von Parametern der Lungenfunktionsprüfung sowie eine Ermittlung von Parametern der Gasanalyse innerhalb eines Messzyklus ermöglicht und durch eine gekoppelte Auswertung die systematische Genauigkeit der Verfahren im Vergleich zu Einzelmessungen erhöht werden. Erfindungsgemäß wird daher ein Verfahren unter Verwendung der Vorrichtung vorgeschlagen, wie in Anspruch 1 definiert. Weiterhin wird vorgeschlagen, dass die Gasmengen-Messvorrichtung eine Gasenergiemaschine umfasst, die sowohl als Arbeitsmaschine als auch als Kraftmaschine einsetzbar ist. Unter einer "Gasenergiemaschine" soll in diesem Zusammenhang insbesondere eine Strömungsmaschine verstanden werden, bei der eine Energieübertragung zwischen dem Gas und der Strömungsmaschine in einem offenen Gasraum stattfindet. Wenn die Gasenergiemaschine als Arbeitsmaschine eingesetzt ist, wird Energie von der Strömungsmaschine an das Gas übertragen. Wenn die Gasenergiemaschine als Kraftmaschine eingesetzt ist, wird Energie aus dem Gas in die Strömungsmaschine übertragen. Die dabei gewonnene Energie wird nur für die Rotation der Strömungsmaschine genutzt. Die Rotationsgeschwindigkeit gibt wiederum Information über den Volumenfluss, der durch die Gasenergiemaschine gedrückt wird.

Die Gasenergiemaschine kann dadurch sowohl zur Bestimmung einer durchströmenden Gasmenge als auch zu einer Unterstützung einer Beatmung eines zu untersuchenden Patienten mittels Reduktion eines effektiven Strömungswiderstands in der Messvorrichtung dienen.

Die Gasenergiemaschine kann vorteilhaft zumindest ein auf einer zentralen Welle angeordnetes Flügelrad umfassen, das in einem Betrieb der Gasenergiemaschine als Arbeitsmaschine als Ventilator und in einem Betrieb der Gasenergiemaschine als Kraftmaschine als Mess-Turbine (Gas-Volumenstromsensor) einsetzbar ist.

Zudem wird vorgeschlagen, dass die Vorrichtung eine Gasaufbereitungsvorrichtung zur Veränderung der chemischen Zusammensetzung des Atemgases aufweist. Dadurch können Möglichkeiten zur Gasanalyse erheblich erweitert oder die Ausführung der Gasanalyse vereinfacht werden.

Wenn die Vorrichtung einen Gas-Volumenstromsensor aufweist, kann eine Bestimmung einer pro Zeiteinheit durch die Vorrichtung strömenden Gasmenge auf eine konstruktiv besonders einfache Weise erreicht werden.

Des Weiteren wird vorgeschlagen, dass die Verbringung zwischen der ersten Ventilpfadkombination und der zweiten Ventilpfadkombination in Abhängigkeit von einer vorbestimmten Zeitdauer oder einer vorbestimmten durchströmten Gasmenge ausführbar ist. Dadurch kann eine Durchführung einer kombinierten Atemgasanalyse und Lungenfunktionsprüfung individuell an Patientenverhältnisse angepasst werden.

Ein weiterer Gegenstand der vorliegenden Offenbarung ist ein Mehrwegeventil zur Verwendung in einer Vorrichtung zur kombinierten Atemgasanalyse und Lungenfunktionsprüfung gemäß einer der zuvor offenbarten Aus- und Weiterbildungen oder einer Kombination davon.

Bei geeigneter Ausgestaltung können Dichtheits- und Kontaminationsprobleme vermieden oder zumindest verringert werden.

Es wird vorgeschlagen, dass die erste Ventilpfadkombination und die zweite Ventilpfadkombination einen gemeinsamen Gaseinlassbereich und einen gemeinsamen Gasauslassbereich aufweisen. Dadurch kann bei geeigneter Ausgestaltung eine Anzahl notwendiger Mehrwegeventile der Vorrichtung verringert werden.

In einer bevorzugten Ausführungsform weist das Mehrwegeventil eine Steuereinrichtung auf, die genau eine Nockenwelle umfasst, wobei die Verbringung zwischen der ersten Ventilpfadkombination und der zweiten Ventilpfadkombination durch eine reversible Drehung der Nockenwelle ausführbar ist. Dabei kann die Verbringung durch eine direkte oder indirekte Wirkung von zumindest zwei Nocken der Nockenwelle auf den ersten oder zweiten Gaspfad erfolgen. Durch die Beschränkung auf genau eine Nockenwelle benötigt das Mehrwegeventil nur einen Antrieb und kann daher besonders kompakt und teilesparend ausgeführt werden.

Wenn die reversible Drehung der Nockenwelle mittels eines Schrittmotors erfolgt, können bistabile und stromlose Funktionsstellungen des Mehrwegeventils erzielt werden.

Außerdem wird vorgeschlagen, dass die Steuervorrichtung zumindest eine erste elastische Membran und eine zweite elastische Membran umfasst, die zwischen der Nockenwelle und einer der zumindest zwei Ventilpfadkombinationen angeordnet und dazu vorgesehen sind, mit jeweils zumindest einer Nocke der Nockenwelle zusammenzuwirken, um die Verbringung zwischen der ersten Ventilpfadkombination und der zweiten Ventilpfadkombination zu erreichen. Dadurch kann die Verbringung zwischen der ersten Ventilpfadkombination und der zweiten Ventilpfadkombination auf eine konstruktiv besonders einfache und kontaminationsfreie Weise erreicht werden.

Unter einer "elastischen Membran" soll in diesem Zusammenhang insbesondere ein im Wesentlichen gasundurchlässiger, als dünne Folie ausgeführter Körper verstanden werden, der im Wesentlichen aus einem federelastischen Material besteht. Insbesondere kann das federelastische Material von einem Elastomer oder einem federelastischen Metall gebildet sein.

In einer besonders vorteilhaften Ausgestaltung sind die erste elastische Membran und die zweite elastische Membran einstückig ausgeführt. Dadurch kann die Konstruktion des Mehrwegeventils besonders einfach ausgeführt sein.

Die Erfindung betrifft die Bereitstellung eines Verfahrens zur kombinierten Atemgasanalyse und Lungenfunktionsprüfung mittels einer Vorrichtung zur kombinierten Atemgasanalyse und Lungenfunktionsprüfung gemäß einer der zuvor offenbarten Aus- und Weiterbildungen oder einer Kombination davon.

Das erfindungsgemäße Verfahren umfasst folgende Schritte:
1. Herstellen des ersten Gaspfads,
2. Einatmen durch Einströmen von Luft eines Außenraums durch die zweite kombinierte Einlass-/Auslassöffnung entlang des ersten Gaspfads in Richtung zur ersten kombinierten Einlass-/Auslassöffnung,
3. Ausatmen zur Lungenfunktionsprüfung zum Durchströmen von Atemgas durch die Gasmengen-Messvorrichtung entlang des ersten Gaspfads von der ersten kombinierten Einlass-/Auslassöffnung in Richtung zur zweiten kombinierten Einlass/Auslassöffnung,
4. Herstellen des zweiten Gaspfads während der Durchführung des Schrittes 3,
5. Durchleiten des durchströmenden Atemgases entlang des zweiten Gaspfads durch die Gasanalysevorrichtung in Richtung zur zweiten kombinierten Einlass-/Auslassöffnung, und
6. Spülen der Gasanalysevorrichtung durch Durchleiten von Luft des Außenraums von der zweiten kombinierten Einlass/Auslassöffnung entlang des zweiten Gaspfads durch die Gasanalysevorrichtung in Richtung zur ersten kombinierten Einlass-/Auslassöffnung.

Durch das Verfahren kann eine Ermittlung von Parametern der Lungenfunktionsprüfung sowie eine Ermittlung von Parametern der Gasanalyse innerhalb eines Messzyklus ermöglicht und durch eine gekoppelte Auswertung die systematische Genauigkeit der Verfahren im Vergleich zu Einzelmessungen erhöht werden.

In einer weiteren vorteilhaften Ausgestaltung kann das Verfahren eine mehrfache Ausführung der Schritte 1 bis 5 vor einer Durchführung des Schritts 6 beinhalten.

### Zeichnung

Weitere Vorteile ergeben sich aus der folgenden Zeichnungsbeschreibung. In der Zeichnung ist ein Ausführungsbeispiel der Vorrichtung und des Mehrwegeventils dargestellt. Die Zeichnung, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen.

### Es zeigen:

Fig. 1 eine schematische Darstellung einer Vorrichtung zur kombinierten Atemgasanalyse und Lungenfunktionsprüfung in einem Betriebszustand einer Einatmungs-/Beatmungsphase,
Fig. 2 eine schematische Darstellung der Vorrichtung gemäß der Fig. 1 in einem Betriebszustand einer Lungenfunktionsprüfungsphase,
Fig. 3 eine schematische Darstellung der Vorrichtung gemäß der Fig. 1 in einem dritten Betriebszustand einer Atemgasanalyse-Phase,
Fig. 4 eine schematische Darstellung der Vorrichtung gemäß der Fig. 1 in einem vierten Betriebszustand einer Spülungsphase,
Fig. 5 eine schematisierte Schnittansicht eines Mehrwegeventils der Vorrichtung gemäß der Fig. 1 in einem Zustand einer ersten Ventilpfadkombination,
Fig. 6 das Mehrwegeventil gemäß der Fig. 5 in gleicher Ansicht in einem Zustand einer zweiten Ventilpfadkombination,
Fig. 7 eine schematisierte Darstellung der pneumatischen Verbindungen des Mehrwegeventils gemäß der Figuren 5 und 6,
Fig. 8 eine erste Ausführungsform einer Nockeneinheit einer Nockenwelle des Mehrwegeventils gemäß der Figuren 5 und 6,
Fig. 9 eine zweite Ausführungsform einer Nockeneinheit der Nockenwelle des Mehrwegeventils gemäß der Figuren 5 und 6, und
Fig. 10 eine alternative Ausführungsform eines Mehrwegeventils in einer schematisierten, perspektivischen Ansicht.

### Beschreibung des Ausführungsbeispiels

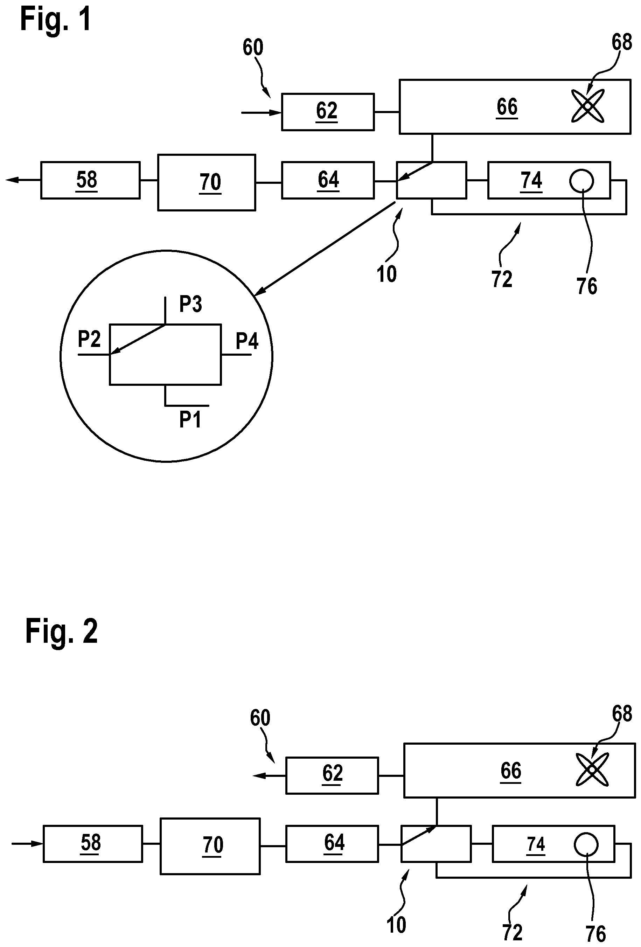

Fig. 1 zeigt eine Vorrichtung zur kombinierten Atemgasanalyse und Lungenfunktionsprüfung in einer schematischen Darstellung. Die Vorrichtung umfasst eine erste kombinierte Einlass-/Auslassöffnung 58 und eine zweite kombinierte Einlass-/Auslassöffnung 60.

Die erste kombinierte Einlass-/Auslassöffnung 58 ist als Mundstück ausgebildet und dazu vorgesehen, von einem Probanden mit dem Außenraum zugewandten Ende am Mund angesetzt zu werden, um ein leichtes Einatmen durch die Vorrichtung und ein leichtes Ausatmen in die Vorrichtung zu ermöglichen.

Die zweite kombinierte Einlass-/Auslassöffnung 60 ist als eine dem Außenraum zugewandte Zugangsöffnung von zwei Zugangsöffnungen eines Aktivkohlefilters 62 ausgebildet.

Ferner umfasst die Vorrichtung eine Gasaufbereitungsvorrichtung 64 zur Veränderung der chemischen Zusammensetzung des Atemgases, bei der Stickstoffmonoxid NO aus dem Atemgas zu Stickstoffdioxid NO₂ umgewandelt wird. Die Gasaufbereitungsvorrichtung 64 ist mit einem dem Außenraum abgewandten Ende des Mundstücks pneumatisch verbunden.

Weiterhin weist die Vorrichtung eine erste Gasmengen-Messvorrichtung 66 zur Bestimmung einer durchströmenden Menge des Atemgases auf, die eine als Lüfter 68 ausgebildete Gasenergiemaschine umfasst. Der Lüfter 68 ist als Kraftmaschine einsetzbar, um eine Menge eines die Vorrichtung durchströmenden Gasstroms zu bestimmen. Der Lüfter 68 ist außerdem als Arbeitsmaschine einsetzbar, um bei Probanden mit erschwerter Atmung ein Einatmen zu erleichtern. Dabei soll hier ein effektiver Strömungswiderstand in der Messvorrichtung reduziert werden.

Für diesen Fall kann die Vorrichtung eine zweite, als Gas-Massenstromsensor ausgebildete Gasmengen-Messvorrichtung 70 zum pneumatischen Anschluss zwischen dem Mundstück und der Gasaufbereitungsvorrichtung 64 umfassen.

Des Weiteren beinhaltet die Vorrichtung eine Gasanalysevorrichtung 72 zur Analyse des Atemgases. Die Gasanalysevorrichtung 72 umfasst eine Messkammer 74 mit einem darin angeordneten Atemanalyse-Sensor 76.

Als zentrale Komponente umfasst die Vorrichtung ein Mehrwegeventil 10. Das Mehrwegeventil 10 weist vier pneumatische Anschlüsse P1, P2, P3, P4 auf. Ein erster pneumatischer Anschluss P1 und ein vierter pneumatischer Anschluss P4 des Mehrwegeventils 10 sind mit je einem Ende der Messkammer 74 pneumatisch verbunden. Ein zweiter pneumatischer Anschluss P2 des Mehrwegeventils 10 ist mit einem dem Mundstück abgewandten Ende der Gasaufbereitungsvorrichtung 64 pneumatisch verbunden. Ein dritter pneumatischer Anschluss P3 des Mehrwegeventils 10 ist mit einem dem Aktivkohlefilter 62 abgewandten Ende der ersten Gasmengen-Messvorrichtung 66 pneumatisch verbunden.

Das Mehrwegeventil 10 ist zwischen einer ersten Ventilpfadkombination A und einer zweiten Ventilpfadkombination B reversibel verbringbar. In der Fig. 1 ist das Mehrwegeventil 10 in der ersten Ventilpfadkombination A dargestellt, mit der unter Umgehung der Gasanalysevorrichtung 72 ein erster Gaspfad zwischen der ersten kombinierten Einlass-/Auslassöffnung 58, der ersten Gasmengen-Messvorrichtung 66 und der zweiten kombinierten Einlass-/Auslassöffnung 60 hergestellt ist.

In dieser Anordnung ist die Vorrichtung auf einen Betriebszustand einer Einatmungs-/Beatmungsphase vorbereitet: ein Weg der Gasströmung verläuft von dem Außenraum durch den Aktivkohlefilter 62, die erste Gasmengen-Messvorrichtung 66, das Mehrwegeventil 10, die Gasaufbereitungsvorrichtung 64 und die zweite Gasmengen-Messvorrichtung 70 über das Mundstück in die Lunge des Probanden. Der Lüfter 68 kann zur Unterstützung der Beatmung mittels Reduzierung des effektiven Strömungswiderstandes der Vorrichtung als Arbeitsmaschine betrieben werden. Die Messkammer 74 befindet sich pneumatisch außerhalb des ersten Gaspfades.

Das Einatmen erfolgt in der Einatmungs-/Beatmungsphase durch Einströmen von Luft des Außenraums durch die zweite kombinierte Einlass-/Auslassöffnung 60 entlang des ersten Gaspfads in Richtung zur ersten kombinierten Einlass-/Auslassöffnung 58.

Fig. 2 zeigt eine schematische Darstellung der Vorrichtung gemäß der Fig. 1 in einem Betriebszustand einer Lungenfunktionsprüfungsphase. Das Mehrwegeventil 10 befindet sich weiterhin in der ersten Ventilpfadkombination A, mit der der erste Gaspfad hergestellt ist.

Das Ausatmen zur Lungenfunktionsprüfung erfolgt durch Durchströmen von Atemgas aus der Lunge des Probanden durch das Mundstück, durch die zweite Gasmengen-Messvorrichtung 70, die Gasaufbereitungsvorrichtung 64, die erste Gasmengen-Messvorrichtung 66 und den Aktivkohlefilter 62 entlang des ersten Gaspfads von der ersten kombinierten Einlass-/Auslassöffnung 58 in Richtung zur zweiten kombinierten Einlass/Auslassöffnung 60.

Bei einer Atemgasanalyse erfolgt die Einatmung im Betriebszustand einer Einatmungs-/Beatmungsphase ähnlich wie bei der Lungenfunktionsprüfung. Das Ausatmen zur Atemgasanalyse erfolgt in der ersten Phase durch Durchströmen von Atemgas aus der Lunge des Probanden durch das Mundstück, durch die zweite Gasmengen-Messvorrichtung 70, die Gasaufbereitungsvorrichtung 64, die erste Gasmengen-Messvorrichtung 66 und den Aktivkohlefilter 62 entlang des ersten Gaspfads von der ersten kombinierten Einlass-/Auslassöffnung 58 in Richtung zur zweiten kombinierten Einlass/Auslassöffnung 60.

In einer zweiten Phase der Atemgasanalyse erfolgt durch Ansteuerung eine Verbringung des Mehrwegeventils 10 von der ersten Ventilpfadkombination A zur zweiten Ventilpfadkombination B, mit der ein zweiter Gaspfad zwischen der ersten kombinierten Einlass-/Auslassöffnung 58, der zweiten Gasmengen-Messvorrichtung 70, der Gasaufbereitungsvorrichtung 64, der Gasanalysevorrichtung 72, der ersten Gasmengen-Messvorrichtung 66, dem Aktivkohlefilter 62 und der zweiten kombinierten Einlass-/Auslassöffnung 60 hergestellt ist. Die Verbringung des Mehrwegeventils 10 in die zweite Ventilpfadkombination B ist dabei wahlweise in Abhängigkeit von einer vorbestimmbaren Zeitdauer oder einer vorbestimmbaren durchströmten Gasmenge ausführbar.

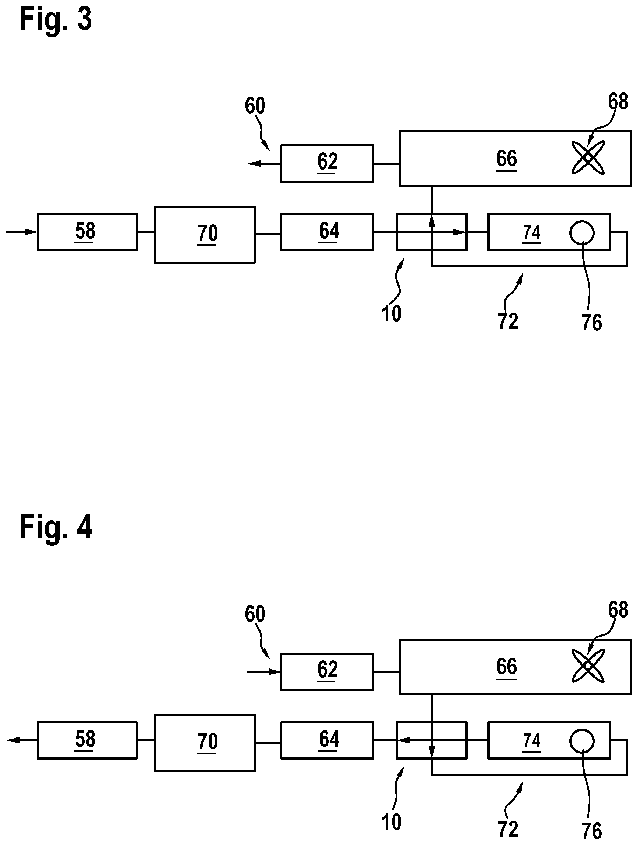

In diesem Betriebszustand, der in der Fig. 3 dargestellt ist, ist die Vorrichtung für die Durchführung einer Atemgasanalyse-Phase vorbereitet. Dabei wird das durchströmende Atemgas entlang des zweiten Gaspfads von der ersten kombinierten Einlass-/Auslassöffnung 58 durch die Gasanalysevorrichtung 72 in Richtung zur zweiten kombinierten Einlass-/Auslassöffnung 60 geleitet.

Nach Beendigung der Atemgasanalyse-Phase, die dem Probanden durch ein akustisches Signal eines nicht dargestellten Steuergerät angezeigt wird, erfolgt ein Spülen der Gasanalysevorrichtung 72 durch Durchleiten von Luft des Außenraums von der zweiten kombinierten Einlass/Auslassöffnung 60 entlang des zweiten Gaspfads durch die Gasanalysevorrichtung 72 in Richtung zur ersten kombinierten Einlass-/Auslassöffnung 58, wobei der Lüfter 68 als Arbeitsmaschine eingesetzt wird. In der Fig. 4 ist die Vorrichtung im Betriebszustand der Spülungsphase in schematischer Weise dargestellt.

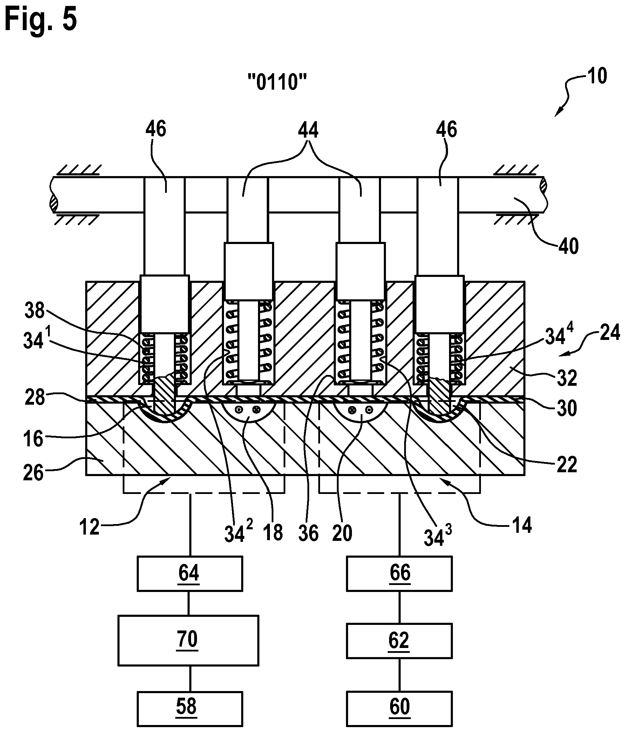

Anhand der Figuren 5 bis 10 sollen Aufbau und Funktion des Mehrwegeventils 10 als zentrale Komponente der Vorrichtung gemäß der Fig. 1 erläutert werden.

Die Fig. 5 zeigt eine schematisierte Schnittansicht des Mehrwegeventils 10 der Vorrichtung gemäß der Fig. 1 in einem Zustand der ersten Ventilpfadkombination A.

Das Mehrwegeventil 10 weist einen Gaseinlassbereich 12 und einen Gasauslassbereich 14 auf. Der Gaseinlassbereich 12 wird im Innenraum des Mehrwegeventils 10 in einem ersten Kanal 16 und einem zweiten Kanal 18 fortgeführt, die in der Nähe des Gaseinlassbereichs 12 voneinander getrennt in einem Kanalblock 26 angeordnet sind. Der Gasauslassbereich 14 wird im Innenraum des Mehrwegeventils 10 ebenfalls in einem dritten Kanal 20 und einem vierten Kanal 22 fortgeführt, die in der Nähe des Gasauslassbereichs 14 voneinander getrennt im Kanalblock 26 verlaufen.

Zur Verbringung des Mehrwegeventils 10 zwischen der ersten Ventilpfadkombination A und der zweiten Ventilpfadkombination B dient eine Steuervorrichtung 24. Die Steuervorrichtung 24 umfasst eine erste elastische Membran 28, die als Abdeckung der beiden Kanäle 16, 18 des Gaseinlassbereichs 12 dient, und eine zweite elastische Membran 30, die als Abdeckung der beiden Kanäle 20, 22 des Gasauslassbereichs 14 dient. Die erste elastische Membran 28 und die zweite elastische Membran 30 sind einstückig ausgeführt, können grundsätzlich aber auch als separate Membranen ausgestaltet sein.

Die erste elastische Membran 28 und die zweite elastische Membran 30 sind auf der dem Kanalblock 26 abgewandten Seite von einem Deckblock 32 abgedeckt, der mit vier senkrecht zu den elastischen Membranen 28, 30 angeordneten, zylindrischen Führungen 34¹ - 34⁴ ausgestattet ist, wobei jeweils eine der Führungen 34¹ - 34⁴ oberhalb eines der Kanäle 16, 18, 20, 22 angeordnet ist. Jede der Führungen 34¹ - 34⁴ weist an einem den elastischen Membranen 28, 30 zugewandten Ende ein Widerlager 36 zur Abstützung eines als Schraubenfeder 38 ausgestalteten Federelements auf.

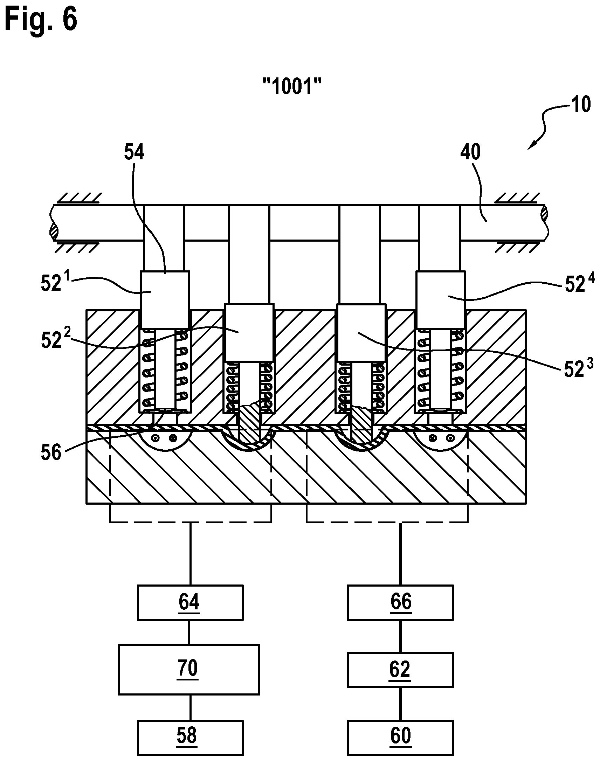

Die Steuervorrichtung 24 umfasst genau eine Nockenwelle 40 mit vier an der Nockenwelle 40 angeordneten Nockeneinheiten 44, 46. Die Nockenwelle 40 ist oberhalb des Deckblocks 32 und parallel zu den beiden elastischen Membranen 28, 30 angeordnet. Unterhalb der Nockenwelle 40 sind vier als Einzelstift ausgebildete Stößel 52¹ - 52⁴ angeordnet (Fig. 6). Jeder der zylindrischen Stößel 52¹ - 52⁴ weist ein erstes, als Kopfelement mit einem an den Durchmesser der Führung 34 angepassten Ende 54 und ein zweites, an einen Innendurchmesser der Schraubenfeder 38 angepasstes Ende 56 sowie eine Erstreckungsrichtung zwischen den beiden Enden 54, 56 auf, die in einem betriebsbereiten Zustand senkrecht zu den beiden elastischen Membranen 28, 30 ausgerichtet ist. Die Stößel 52¹ - 52⁴ sind dazu vorgesehen, in den Führungen 34¹ - 34⁴ des Deckblocks 32 lineare Bewegungen parallel zu der jeweiligen Erstreckungsrichtung auszuführen.

Im betriebsbereiten Zustand gelangen die Oberseiten der Kopfenden der Stößel 52¹ - 52⁴ aufgrund der Federkraft der Schraubenfedern 38 mit den Nocken 48¹ - 48⁴, 50¹ - 50⁴ der Nockeneinheiten 44, 46 in federbelastete Anlage.

Die zweiten Enden 56 der Stößel 52¹ - 52⁴ sind mit einem Krümmungsradius ausgestattet, der an die Querschnittsform der Kanäle 16, 18, 20, 22 angepasst ist. Die Stößel 52¹ - 52⁴ sind dazu vorgesehen, mittels einer der Nocken 48¹ - 48⁴, 50¹ - 50⁴ der Nockenwelle 40 gegen die Federkraft einer der Schraubenfedern 38 innerhalb einer der Führungen 34¹ - 34⁴ des Deckblocks 32 parallel zur Erstreckungsrichtung eine lineare Bewegung in Richtung auf eine der beiden elastischen Membranen 28, 30 auszuführen, um mittels des zweiten Endes 56 des Stößels 52¹ - 52⁴ und aufgrund der elastischen Eigenschaft der Membranen 28, 30 jeweils einen der Kanäle 16, 18, 20, 22 zu verschließen. Durch diese Ausführung ist erreicht, dass ein zur Leitung des Atemgases vorgesehener Fluidraum pneumatisch vollständig dicht ist und ein direkter Kontakt zu den Stößeln 52¹ - 52⁴ der Steuervorrichtung 24 vermieden werden kann.

Auf einer dem Gaseinlassbereich 12 bzw. dem Gasauslassbereich 14 des Mehrwegeventils 10 abgewandten Seite sind der zweite Kanal 18 und der dritte Kanal 20 pneumatisch miteinander verbunden. Ferner sind der erste Kanal 16 auf einer dem Gaseinlassbereich 12 abgewandten Seite mit dem einen Ende der Messkammer 74 und der vierte Kanal 22 auf einer dem Gasauslassbereich 14 abgewandten Seite mit dem anderen Ende der Messkammer 74 pneumatisch verbunden.

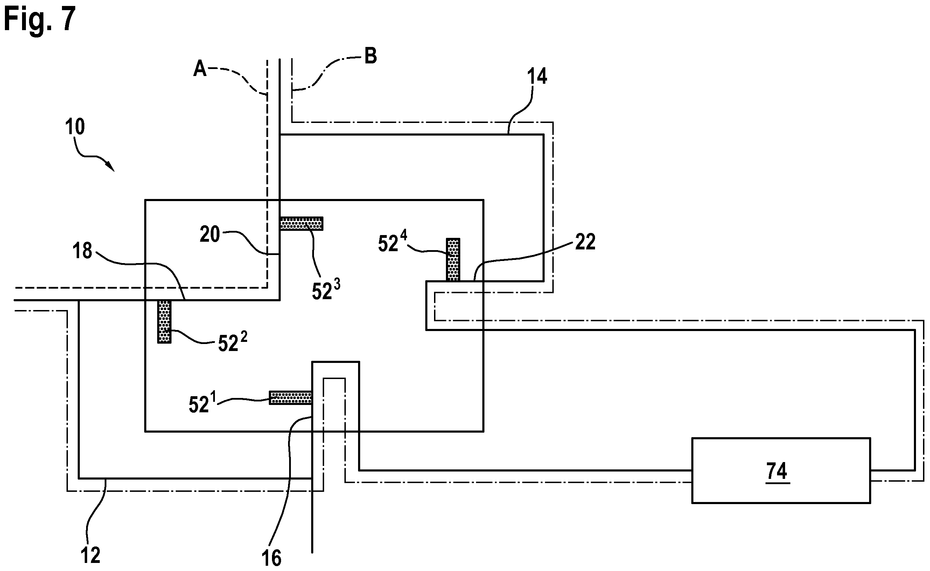

Zur Verdeutlichung sind in Fig. 7 in einer schematisierten Darstellung die pneumatischen Verbindungen der Kanäle 16, 18, 20, 22 und die Anordnung der Stößel 52¹, 52⁴ innerhalb des Mehrwegeventils 10 wiedergegeben.

Die in der Fig. 5 dargestellte erste Ventilpfadkombination A ist dadurch gekennzeichnet, dass die Nocken 48¹ - 48⁴, 50¹- 50⁴ der Nockenwelle 40 und deren Rotationsposition so ausgelegt sind, dass der erste Kanal 16 mittels des ersten Stößels 52¹ und der ersten Membran 28 und der vierte Kanal 22 mittels des vierten Stößels 52⁴ und der zweiten Membran 30 verschlossen sind und der zweite Kanal 18 und der dritte Kanal 20 geöffnet sind.

Die in der Fig. 5 gezeigte erste Ventilpfadkombination A ist in der Fig. 7 durch eine gestrichelte Linie angezeigt.

Fig. 6 zeigt eine schematisierte Schnittansicht des Mehrwegeventils 10 in einem Zustand der zweiten Ventilpfadkombination B. Eine Verbringung des Mehrwegeventils 10 von der ersten Ventilpfadkombination A zur zweiten Ventilpfadkombination B ist durch eine reversible Drehung der Nockenwelle 40 ausführbar.

Die zweite Ventilpfadkombination B ist dadurch gekennzeichnet, dass die Nocken 48¹ - 48⁴, 50¹ - 50⁴ der Nockenwelle 40 und deren Rotationsposition so ausgelegt sind, dass der zweite Kanal 18 mittels des zweiten Stößels 52² und der ersten Membran 28 und der dritte Kanal 20 mittels des dritten Stößels 52³ und der zweiten Membran 30 verschlossen sind und der erste Kanal 16 und der vierte Kanal 22 geöffnet sind.

Die in der Fig. 6 gezeigte zweite Ventilpfadkombination B ist in der Fig. 7 durch eine strichpunktierte Linie angezeigt. Wie aus Fig. 7 ersichtlich weisen die erste Ventilpfadkombination A und die zweite Ventilpfadkombination B den gemeinsamen Gaseinlassbereich 12 und den gemeinsamen Gasauslassbereich 14 auf.

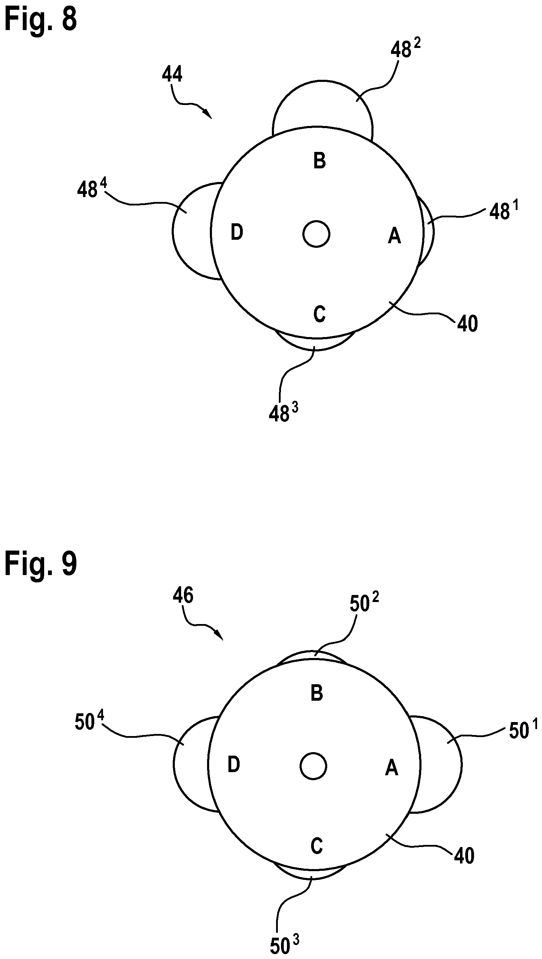

Fig. 8 zeigt eine Ausführungsform der Nockeneinheit 44 der Nockenwelle 40, wobei je eine Nockeneinheit 44 zur Ansteuerung des zweiten Stößels 52² und des dritten Stößels 52³ vorgesehen ist. Vier Nocken 48¹-48⁴ sind in der Nockeneinheit 44 in einem gleichmäßigen Winkelabstand von 90° bezüglich der Nockenwelle 40 angeordnet, so dass eine Verbringung des Mehrwegeventils 10 von einer Ventilpfadkombination A, B, C, D zu einer anderen Ventilpfadkombination A, B, C, D durch eine reversible Drehung der Nockenwelle 40 um 90° ausführbar ist, wobei vier verschiedene Stellungen der Nockenwelle 40 entsprechend den vier verschiedenen Ventilpfadkombinationen A, B, C, D des Mehrwegeventils 10 möglich sind. Die Nocken 48¹-48⁴ zur Ansteuerung des zweiten Stößels 52² und des dritten Stößels 52³ können alternativ auch einstückig ausgeführt sein.

Fig. 9 zeigt eine Ausführungsform der Nockeneinheit 46 der Nockenwelle 40, wobei je eine Nockeneinheit 46 zur Ansteuerung des ersten Stößels 52¹ und des vierten Stößels 52⁴ vorgesehen ist. Auch hier sind vier Nocken 50¹-50⁴ in einem gleichmäßigen Winkelabstand von 90° bezüglich der Nockenwelle 40 angeordnet.

Die in den Figuren 8 und 9 dargestellten flacheren Nocken 48¹, 48³, 50², 50³ ermöglichen den darunter angeordneten Stößeln 52¹ - 52⁴, von der Federkraft der entsprechenden Schraubenfeder 38 getrieben von den elastischen Membranen 28, 30 abzuheben und den unter den elastischen Membranen 28, 30 liegenden Kanal 16, 18, 20, 22 freizugeben. Die in den Figuren 8 und 9 dargestellten höheren Nocken 48², 48⁴, 50¹, 50⁴ sind dazu ausgelegt, die darunter angeordneten Stößel 52¹ - 52⁴ gegen die Federkraft der Schraubenfeder 38 zu einer linearen Bewegung in Richtung auf die elastische Membran 28, 30 zwangszuführen und den unter der elastischen Membran 28, 30 liegenden Kanal 16, 18, 20, 22 zu verschließen. Die zwischen der Nockenwelle 40 und den Ventilpfadkombinationen A, B, C, D angeordnete erste elastische Membran 28 und zweite elastische Membran 30 sind dazu vorgesehen, mit jeweils zumindest einer der Nocken 48¹ - 48⁴, 50¹-50⁴ der Nockenwelle 40 zusammenzuwirken, um die Verbringung des Mehrwegeventils 10 zwischen einer ersten der Ventilpfadkombinationen A, B, C, D und einer zweiten der Ventilpfadkombinationen A, B, C, D zu erreichen.

Die reversible Drehung der Nockenwelle 40 kann von Hand erfolgen. Sie kann alternativ aber auch mittels eines nicht dargestellten Schrittmotors erreicht werden, wobei mehrfach-stabile und stromlose Ventilpfadkombinationen A, B, C, D des Mehrwegeventils 10 erreichbar sind.

Die möglichen Stellungen der Stößel 52¹ - 52⁴ können zur Vereinfachung durch Angabe einer binären Zahl bezeichnet werden. Dabei soll die binäre Zahl "0" eine Stellung eines Stößels 52¹ - 52⁴ bezeichnen, in der ein unter dem Stößel 52¹ - 52⁴ angeordneter Kanal 16, 18, 20, 22 verschlossen ist. Die binäre Zahl "1" soll einen offenen Kanal 16, 18, 20, 22 bezeichnen.

Die in der Fig. 5 gezeigte erste Ventilpfadkombination A kann demnach durch die binäre Zahl "0110" bezeichnet werden. Die in der Fig. 6 gezeigte zweite Ventilpfadkombination B entspricht der binären Zahl "1001".

Mit den in den Figuren 8 und 9 dargestellten Nocken 48¹ - 48⁴, 50¹ - 50⁴ der Nockeneinheiten 44, 46 ergeben sich folgende weitere Ventilpfadkombinationen:
- C - "1111" (alle Kanäle 16, 18, 20, 22 geöffnet; Wartungsstellung)
- D - "0000" (alle Kanäle 16, 18, 20, 22 geschlossen; Lagerungsstellung)

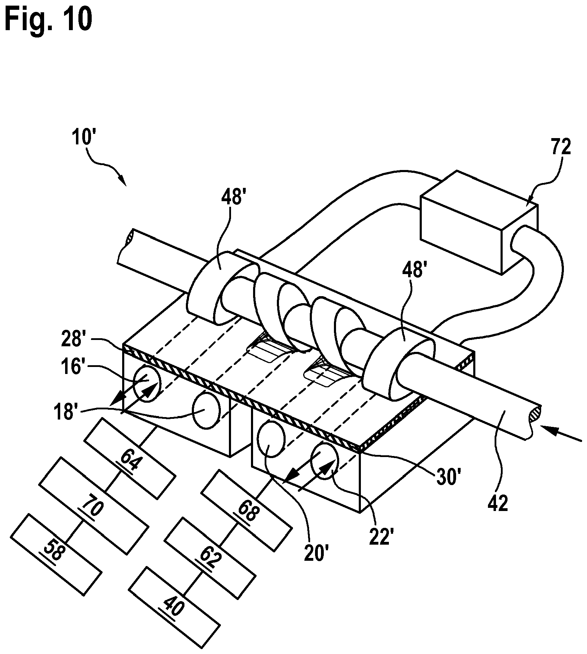

Fig. 10 zeigt eine alternative Ausführungsform eines Mehrwegeventils 10' in einer schematisierten, perspektivischen Ansicht. Im Unterschied zur ersten Ausführungsform entfallen hierbei die Stößel und der Deckblock mitsamt Führungen und Schraubenfedern, so dass auf einer Nockenwelle 42 angeordnete Nocken 48', 50' dazu vorgesehen sind, in direkter Weise auf eine erste elastische Membran 28' und eine zweite elastische Membran 30' derart mechanisch einzuwirken, dass ein unterhalb des Nockens 48', 50' und einer der Membranen 28', 30' angeordneter Kanal 16', 18', 20', 22' des Mehrwegeventils 10' gasdicht verschließbar ist und durch eine weitere reversible Drehung der Nockenwelle 42 erneut geöffnet werden kann.

## Patentansprüche

1. Verfahren zur kombinierten Atemgasanalyse und Lungenfunktionsprüfung mittels einer Vorrichtung, wobei die Vorrichtung folgende Merkmale umfasst:
- eine erste kombinierte Einlass-/Auslassöffnung (58),
- eine zweite kombinierte Einlass-/Auslassöffnung (60),
- eine Gasmengen-Messvorrichtung (66, 70) zur Bestimmung einer durchströmenden Menge des Atemgases,
- eine Gasanalysevorrichtung (72) zur Analyse des Atemgases,
- ein Mehrwegeventil (10), das zumindest zwischen einer ersten Ventilpfadkombination (A, B, C, D) und einer zweiten Ventilpfadkombination (A, B, C, D) verbringbar ist, wobei
- mit der ersten Ventilpfadkombination (A, B, C, D) des Mehrwegeventils (10) unter Umgehung der Gasanalysevorrichtung (72) ein erster Gaspfad zwischen der ersten kombinierten Einlass-/Auslassöffnung (58), der Gasmengen-Messvorrichtung (66, 70) und der zweiten kombinierten Einlass-/Auslassöffnung (60) herstellbar ist, und
- mit der zweiten Ventilpfadkombination (A, B, C, D) des Mehrwegeventils (10) ein zweiter Gaspfad zwischen der ersten kombinierten Einlass-/Auslassöffnung (58), der Gasanalysevorrichtung (72), der Gasmengen-Messvorrichtung (66, 70) und der zweiten kombinierten Einlass-/Auslassöffnung (60) herstellbar ist,
wobei das Verfahren folgende Schritte umfasst:
- Herstellen des ersten Gaspfads,
- Einatmen durch Einströmen von Luft eines Außenraums durch die zweite kombinierte Einlass-/Auslassöffnung (60) entlang des ersten Gaspfads in Richtung zur ersten kombinierten Einlass-/Auslassöffnung (58),
- Ausatmen zur Lungenfunktionsprüfung zum Durchströmen von Atemgas durch die Gasmengen-Messvorrichtung (66, 70) entlang des ersten Gaspfads von der ersten kombinierten Einlass-/Auslassöffnung (58) in Richtung zur zweiten kombinierten Einlass/Auslassöffnung (60),
- Herstellen des zweiten Gaspfads während der Durchführung des vorhergenannten Schrittes,
- Durchleiten des durchströmenden Atemgases entlang des zweiten Gaspfads durch die Gasanalysevorrichtung (72) in Richtung zur zweiten kombinierten Einlass-/Auslassöffnung (60), und
- Spülen der Gasanalysevorrichtung (72) durch Durchleiten von Luft des Außenraums von der zweiten kombinierten Einlass/Auslassöffnung (60) entlang des zweiten Gaspfads durch die Gasanalysevorrichtung (72) in Richtung zur ersten kombinierten Einlass-/Auslassöffnung (58).

## Claims

1. Method for combined respiratory gas analysis and lung function testing by means of a device, wherein the device comprises the following features:
- a first combined inlet/outlet opening (58),
- a second combined inlet/outlet opening (60),
- a gas quantity measuring device (66, 70) for determining an amount of the respiratory gas flowing through,
- a gas analysis device (72) for analyzing the respiratory gas,
- a multiway valve (10) which can be brought at least between a first valve path combination (A, B, C, D) and a second valve path combination (A, B, C, D), wherein
- with the first valve path combination (A, B, C, D) of the multiway valve (10), while circumventing the gas analysis device (72), a first gas path can be established between the first combined inlet/outlet opening (58), the gas quantity measuring device (66, 70) and the second combined inlet/outlet opening (60), and
- with the second valve path combination (A, B, C, D) of the multiway valve (10), a second gas path can be established between the first combined inlet/outlet opening (58), the gas analysis device (72), the gas quantity measuring device (66, 70) and the second combined inlet/outlet opening (60), wherein the method comprises the following steps:
- establishing the first gas path,
- inhaling by flow of air from an external space through the second combined inlet/outlet opening (60) along the first gas path in the direction of the first combined inlet/outlet opening (58),
- exhaling for lung function testing in order for respiratory gas to flow through the gas quantity measuring device (66, 70) along the first gas path from the first combined inlet/outlet opening (58) in the direction of the second combined inlet/outlet opening (60),
- establishing the second gas path while carrying out the preceding step,
- conducting the respiratory gas, which is flowing through, along the second gas path through the gas analysis device (72) in the direction of the second combined inlet/outlet opening (60), and
- flushing the gas analysis device (72) by conducting air from the external space from the second combined inlet/outlet opening (60) along the second gas path through the gas analysis device (72) in the direction of the first combined inlet/outlet opening (58).

## Revendications

1. Procédé permettant de combiner une analyse des gaz respiratoires et une vérification des fonctions pulmonaires au moyen d'un dispositif, dans lequel le dispositif présente les caractéristiques suivantes:
- une première ouverture d'entrée/sortie combinée (58),
- une deuxième ouverture d'entrée/sortie combinée (60),
- un dispositif de mesure de quantité de gaz (66, 70), pour la détermination d'une quantité en écoulement du gaz respiratoire,
- un dispositif d'analyse de gaz (72) pour l'analyse du gaz respiratoire,
- une vanne à plusieurs voies (10), qui peut être déplacée au moins entre une première combinaison de chemin de vanne (A, B, C, D) et une deuxième combinaison de chemin de vanne (A, B, C, D), dans lequel
- avec la première combinaison de chemin de vanne (A, B, C, D) de la vanne à plusieurs voies (10) avec contournement du dispositif d'analyse de gaz (72), un premier chemin de gaz peut être établi entre la première ouverture d'entrée/sortie combinée (58), le dispositif de mesure de quantité de gaz (66, 70) et la deuxième ouverture d'entrée/sortie combinée (60), et
- avec la deuxième combinaison de chemin de vanne (A, B, C, D) de la soupape à plusieurs voies (10), un deuxième chemin de gaz peut être établi entre la première ouverture d'entrée/sortie combinée (58), le dispositif d'analyse de gaz (72), le dispositif de mesure de quantité de gaz (66, 70) et la deuxième ouverture d'entrée/sortie combinée (60),
dans lequel le procédé comprend les étapes suivantes:
- établissement du premier chemin de gaz,
- inspiration par aspiration d'air d'un espace extérieur à travers la deuxième ouverture d'entrée/sortie combinée (60) le long du premier chemin de gaz en direction de la première ouverture d'entrée/sortie combinée (58),
- expiration pour la vérification des fonctions pulmonaires par écoulement de gaz respiratoire à travers le dispositif de mesure de quantité de gaz (66, 70) le long du premier chemin de gaz de la première ouverture d'entrée/sortie combinée (58) en direction de la deuxième ouverture d'entrée/sortie combinée (60),
- établissement du deuxième chemin de gaz pendant l'exécution de l'étape précédente,
- transfert du gaz respiratoire en écoulement le long du deuxième chemin de gaz à travers le dispositif d'analyse de gaz (72) en direction de la deuxième ouverture d'entrée/sortie combinée (60), et
- purge du dispositif d'analyse de gaz (72) par transfert d'air de l'espace extérieur de la deuxième ouverture d'entrée/sortie combinée (60) le long du deuxième chemin de gaz à travers le dispositif d'analyse de gaz (72) en direction de la première ouverture d'entrée/sortie combinée (58).
